# EUROPEAN PATENT SPECIFICATION

(11) **EP 0 570 682 B1**
(45) Date of publication and mention of the grant of the patent: **23.07.1997**
(21) Application number: 93104369.9
(22) Date of filing: 17.03.1993
(51) Int. Cl.: C07H 13/04, A61K 39/05, A61K 39/08, A61K 47/48, C07H 15/18

(54) **Synthetic lipid A glycoconjugate antigens for use in vaccines**
Synthetische Lipid-A Clykoconjugate-Antigene und deren Verwendung in Impfstoffen
Glycoconjugates synthétiques d'antigènes de lipid A et leur utilisation comme vaccins

(30) Priority: 07.05.1992 US 879403
(43) Date of publication of application: 24.11.1993
(73) Proprietor: AMERICAN CYANAMID COMPANY, Wayne, NJ 07470-8426 (US)
(72) Inventor: Porro, Massimo, Rapolano Terme, Siena 53040 (IT)
(74) Representative: Wächtershäuser, Günter, Prof. Dr.

(56) References cited:
- EP-A- 0 208 375
- WO-A-91/01750
- BIOCHEMISTRY vol. 24, 1985, pages 2736 - 2742 D. CHARON ET AL. 'Chemical Synthesis and Immunological Activities of Glycolipids Structurally Related to Lipid A' *
- J. BIOL. CHEM. vol. 266, 1991, pages 4719 - 4725 R. DZIARSKI 'Peptidoglycan and Lipopolysaccharide Bind to the Same Binding Site on Lymphocytes' *

## Description

### 1. Field of the Invention

The present invention relates to synthetic glycoconjugate antigens for use in vaccines for prophylaxis of septic shock caused by bacterial endotoxin and novel methods of preparing the glycoconjugates.

### 2. Background

### A. Septic Shock

Septic shock is a life-threatening condition which occurs following infections by Gram-negative bacteria as complications of surgery, prolonged hospitalization, accidents and other traumatic events. It is well recognized that the agent responsible for this disease is the bacterial endotoxin, a glycolipid antigen present only on the surface of Gram-negative bacteria. This glycolipid is also known as lipo-polysaccharide (LPS) or lipo-oligosaccharide (LOS) depending on the size of the carbohydrate chain which is covalently bound to the fatty-acid-rich moiety called Lipid A (LipA). Only Lipid A is responsible for the major toxic effects shown by endotoxin (LPS). The release of this endotoxin which is anchored on the outer membrane of the bacterium, to the blood-stream, results in binding to specialized cells of the immune system like macrophages and monocytes. These specialized cells are in turn activated by the endotoxin and several immune mediators are released (Interleukin-1 and Interleukin-6; α-Tumor necrosis factor; δ-Interferon). Furthermore, endotoxin also activates the complement cascade which results in cell lysis with the consequent release of proteolytic enzymes promoting the release of vasoactive effectors from platelets (e.g.: bradykinin and histamine). Alteration of the levels of these cytokines finally results in a sharp fall in blood pressure followed by heart failure. The final result is death of the patient in 40-60% of the cases within 48-72 hours. So far, there has been no specific cure or therapy available although bolus injections of adrenal corticosteroids such as methylprednisolone are used and recently the use of monoclonal antibodies have given some encouraging results.

Septic shock can be caused by infection with any bacteria which cause the release of LPS. These bacteria include Pseudomonas aeroginosa, Escherichea coli, Salmonella typhi, Neisseria gonorrheae, Bordetella pertussis, Klebsiella pneumoniae and the like.

### B. Lipid A and Synthetic Analogs

One of the main reasons for the lack of efficiency of the host's immune system to block endotoxin before triggering the target cells above mentioned, is due to the fact that endotoxin is a very poor antigen, especially in the structure which is involved in the toxic activity, called Lipid A. Lipid A consists of a backbone of β-(1-->6) diglucosamine with several acyl residues (fatty acids) covalently bound to the hydroxyl and amino groups via ester and amido linkages respectively. Four molecules of 3-hydroxytetradecanoate are attached to the glucosamine disaccharide and other hydroxyl groups are substituted with normal fatty acids to form acyloxyacyl groups.

Various synthetic analogs of Lipid A have been prepared in an effort to increase the immunologic activities of Lipid A but eliminate its toxicity.

While other synthetic analogs of Lipid A have been described, the present invention involves synthetic Lipid A analogs with chemical characteristics uniquely tailored for covalent coupling to carrier proteins without affecting their antigenic structrue.

### C. Conjugate Vaccines

In an effort to increase the immunogenicity of polysaccharides that are weak antigens, it is known to prepare immunogenic conjugates where the polysaccharide is coupled to carrier proteins to produce so called "conjugate" vaccines. For example, immune responsiveness in human infants to Haemophilus influenzae b capsular polysaccharide has been achieved by coupling the capsular antigen to a carrier protein of diptheria toxoid. It is believed that lymphocytic helper effects are induced by the carrier protein and are responsible for development of immunity. A similar approach has been directed toward producing pneumococcal vaccines. Intact capsular polymers derived from various bacterial capsular polymers have been used as the antigenic components in these vaccines.

### D. Use of Carrier Proteins to Make Antiserum to Haptens

Carrier proteins can do more than enhance the immunogenicity of conjugated capsular polymers; they can also render haptens immunogenic. Haptens are defined as molecules that can bind specifically to an antibody or lymphocyte receptor but may not themselves induce an immune response (i.e. they are not immunogenic). To evoke an immune response, small/low molecular weight or poorly immunogenic molecules, termed haptens, must generally first be coupled to a larger molecule, or carrier, which is usually a heterologous protein. Injection of the hapten-carrier complex into an animal will then give rise to the production by B lymphocytes of antibodies, some of which will be capable of specifically binding to the free, uncoupled hapten molecule.

Among the earliest haptens to be studied were azo dye compounds such as aniline and 0-aminobenzoic acid. Landsteiner and Lampl (1918, Z. Immun. Forsch 26:293) coupled these compounds by diazotization to serum proteins. When injected with these artifically prepared azo-proteins, rabbits developed precipitating antibodies that were specific for the attached chemical moieties.

Other examples of haptenic compounds are dinitrophenol, which becomes immunogenic upon coupling as the dinitrophenyl (DNP) group to bovine serum albumin or to bovine gamma globulin (BGG), and lysergic acid diethylamide. Even formaldehyde has been shown to behave as a hapten; persons exposed to formaldehyde vapors from products or in laboratories have become "sensitized" to the compound, following the formylation of their endogenous macromolecules in vivo.

Haptenic behavior is not limited to small organic molecules, and polypeptide hormones up to the size of insulin are often poorly, if at all, immunogenic. To obtain high antibody titers to these hormones it is thus necessary to conjugate them to a carrier molecule (or to create larger molecules by crosslinking many of these polypeptides together).

The involvement of the carrier molecule is especially interesting in that the carrier plays more than a mere transport role. Ovary and Benaceraff 1963, Proc. Soc. Exp. Biol. Med. 114:723 showed this by injecting rabbits with DNP-BCG. Injection of many immunogenic materials into animals will produce an immunological "memory" of the exposure. When a second injection is given later, there is thus a much more vigorous immune response. Indeed, when Ovary and Benaceraff injected DNP-BCG again, there was a strong, secondary response that led to markedly elevated levels of antibodies directed against both DNP and BCG. But when the second injection was instead made with DNP-egg albumin, a much weaker anti-DNP antibody response was noted. The difference in response was due to what has been called the carrier effect, and it appears to involve helper T lymphocytes.

Preliminary evidence indicates that all proteins may not be equally effective carrier proteins for a given hapten. Robbins, et al. (Infect. Immun. 40:245-256) have presented data on experimental protein-polysaccharide conjugate vaccines in which the same polysaccharide hapten was conjugated to different protein carriers and the antibody response to the hapten was quantified. Significant differences were noted in the amount of anti-hapten antibody generated, indicating a major role for the carrier.

### E. Vaccines Containing Conjugates

Other researchers have studied conjugation of capsular polymers to carrier proteins in an effort to enhance antibody formation by the so-called "carrier effect". For example, Schneerson et al., Journal of Experimental Medicine 152:361-376 (1980) describes H. influenzae b polymer-protein conjugates disclosed to confer immunity to invasive diseases caused by H. influenzae b. The reference documents the age-related immunological behavior of capsular polymers in infants and seeks to overcome this age-dependence by conjugation of the intact capsular polymer with a variety of proteins, including serum albumins, Limulus polyphemus hemocyanin and diphtheria toxin. The method of conjugation involves the use of a linking agent such as adipic dihydrazide.

Geyer et al., Med. Microbiol. Immunol. 165:171-288 (1979), prepared conjugates of certain Klebsiella pneumoniae capsular polysaccharide fragments to a nitrophenyl-ethylamine linker by reductive amination, and the derivatized sugar was then attached to proteins using azo coupling.

U.S. Patent No. 4,057,685 by McIntire, filed May 9, 1974 relates to an Escherichia coli lipopolysaccharide of reduced toxicity covalently coupled to a protein antigen by reaction with haloacyl halide.

U.S. Patent No. 4,356,170 by Jennings et al., issued October 26, 1982, relates to the production of polysaccharide-protein conjugates by reductive amination.

Anderson (1983, Infection and Immunity 39:233-238) relates to conjugates between oligosaccharides from Haemophilus influenzae type b capsular polysaccharide and CRM₁₉₇, a nontoxic but antigenically identical variant of diphtheria toxin.

Snippe et al., (1983, Infection and Immunity 42:842-844), relates to a semisynthetic vaccine to Streptococcus pneumoniae type 3 in which a hexasaccharide isolated from a partial acid hydrolysate of the capsular polysaccharide S3 was coupled to stearyl amine by reductive amination and then incorporated into liposomes. The resulting conjugate/liposome vaccine was observed to induce protection to S. pneumoniae type 3 in mice.

U.S. Patent No. 4,663,160 by Tsay et al., issued May 5, 1987, relates to bacteria in which a detoxified polysaccharide from a gram-negative bacterium is covalently coupled to a detoxified protein from the same species of gram-negative bacterium, by means of a 4-12 carbon moiety.

U.S. Patent No. 4,619,828 by Gordon, issued October 28, 1986, relates to conjugates between polysaccharide molecules from pathogenic bacteria such as Haemophilus influenzae b, Streptococcus pneumoniae, Neisseria meningitidis, and Escherichia coli and T cell dependent antigens such as diphtheria and tetanus toxoids.

U.S. Patent No. 4,808,700 by Anderson and Clements, issued February 28, 1989, and U.S. Patent No. 4,761,283 by Anderson, issued August 2, 1988, relate to the covalent attachment of capsular polymer fragments to bacterial toxins, toxoids, or binding subunits by means of reductive amination.

U.S. Patent No. 4,711,779 by Porro et al., issued December 8, 1987, relates to glycoprotein conjugate vaccines having trivalent immunogenic activity and comprising antigenic determinants from the capsular polysaccharides of a gram positive bacterium and a gram negative bacterium, as well as either CRM₁₉₇, tetanus toxoid, or pertussis toxin.

### F. Methods for Preparing Conjugate Vaccines

The preparation of conjugate vaccines, in which capsular polysaccharide haptens are linked to carrier proteins, entails the following procedures:
(i) capsular polysaccharide must be prepared;
(ii) if a fragment of the polysaccharide is to be used, it must be separated from intact polysaccharide;
(iii) saccharide must be activated, or rendered amenable to conjugation, i.e. moieties capable of covalently bonding to protein must be generated;
(iv) saccharide is conjugated to protein.
Various methods are known in the art for accomplishing these four steps. For a review of the methods employed, see Cruse JM, Lewis RE Jr. (Eds): Conjugate Vaccines, Contrib. Microbiol. Immunol.; Basel, Karger, 1989, Vol. 10, pp 48-114.

In general, the coupling can be direct or with the use of a linker (spacer) chain which serve to separate the two antigenic components by chains whose length and flexibility can be tailored to need. The chemical reactions employed for the coupling generally depend on use or modification of a relatively small number of naturally occurring functional groups: carboxyl, hemiacetal, amino/imino, mercapto/disulfide, hydroxyl and phenoxyl moieties. For example, glyconjugation can be accomplished by formation of amide bonds utilizing a water soluble carbodiimide with or without a water-soluble hydroxysuccinimide derivative to form activated carboxylate intermediates that readily condense with primary amino groups on the carrier protein. Another method commonly used, termed "reductive amination" utilizes sodium cyanoborohydride to selectively reduce intermediate imine adducts known as Schiff bases. See U.S. Patent Nos. 4,356,170 and 4,808,700. Currently, it is used to produce a commercial PRP-derived oligosaccharide vaccine (HbOC).

A variety of coupling techniques which use linker technology are known. One method uses adipic acid dihydrazide as a linker, coupling with cyanogen bromide. See U.S. Patent No. 4,619,828. In U.S. Patent No. 4,711,779, the oligosaccharide haptens are first cleaved by acid hydrolyses then activated by introducing primary amino groups into the terminal reducing groups (e.g. using sodium cyanoborohydride), with subsequent conversion to esters (e.g. in the presence of adipic acid derivatives). The activated oligosaccharide is then conjugated to toxoid in the presence of organic solvent such as dimethylsulfoxide.

WO-A-91/01750 describes synthetic ester-type glycoconjugates for use in the treatment of septic shock.

Biochemistry, 1985, 24, 2736-42 describes 2,2'-long chain N-acyl derivatives of 1-6 linked 2,2'-deoxy glucosyl glucosides with bacterial endotoxic activity.

J. Biol. Chem. 1991, 266, 4719-25 describes the variability in disaccharide structure with regard to lymphocyte binding.

### SUMMARY OF THE INVENTON

The present invention relates to novel synthetic disaccharide haptens which are analogs of Lipid A and immunogenic conjugates prepared from such disaccharides by covalent coupling of the disaccharides to carrier proteins.

In addition, the present invention relates to novel methods of synthesizing the synthetic disaccharide haptens and producing primary amino groups in the disaccharides which are essential in the conjugation procedure employed in making the immunogenic conjugates.

Further, the present invention relates to novel intermediates useful in preparing the synthetic Lipid A disaccharides.

Finally, the present invention relates to synthetic Lipid A conjugate vaccines and their use in the treatment of septic shock.

Accordingly, the present invention is directed to novel synthetic Lipid A D-Glucosamine disaccharide conjugates of the general formula: 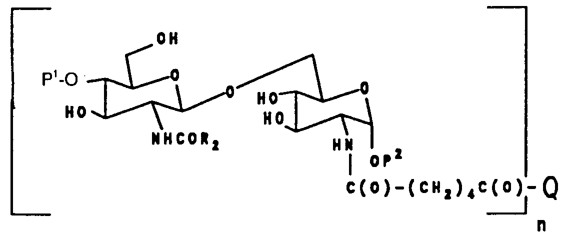 wherein P¹ is hydrogen or phosphate; R₂ is alkyl(C₁-C₄); P² is hydrogen, propyl, allyl or phosphate; n is an integer from 1-30 and Q is a suitable carrier protein or peptide. In the foregoing formula, n expresses the number of moles of disaccharide units per mole of carrier protein.

### DETAILED DESCRIPTION

### 1. Preparation of D-Glucosamine Disaccharides

The novel synthetic disaccharide haptens useful in preparing the immunogenic conjugates of the present invention are those of the formula: 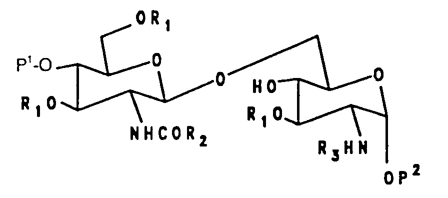 wherein
R₁ is hydrogen or acyl;
R₂ is (C₁-C₄) alkyl;
R₃ is hydrogen or CO(CH₂)₄-COOX wherein X is a functional group of the formula: 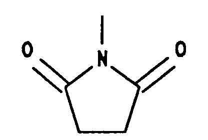
P¹ is hydrogen or phosphate, and P² is hydrogen, propyl, allyl or phosphate.

These novel Lipid A D-glucosamine disaccharides of the present invention which may be represented by compounds 13, 32 and 35 and the novel conjugates thereof may be prepared as outlined in Scheme I and Scheme II.

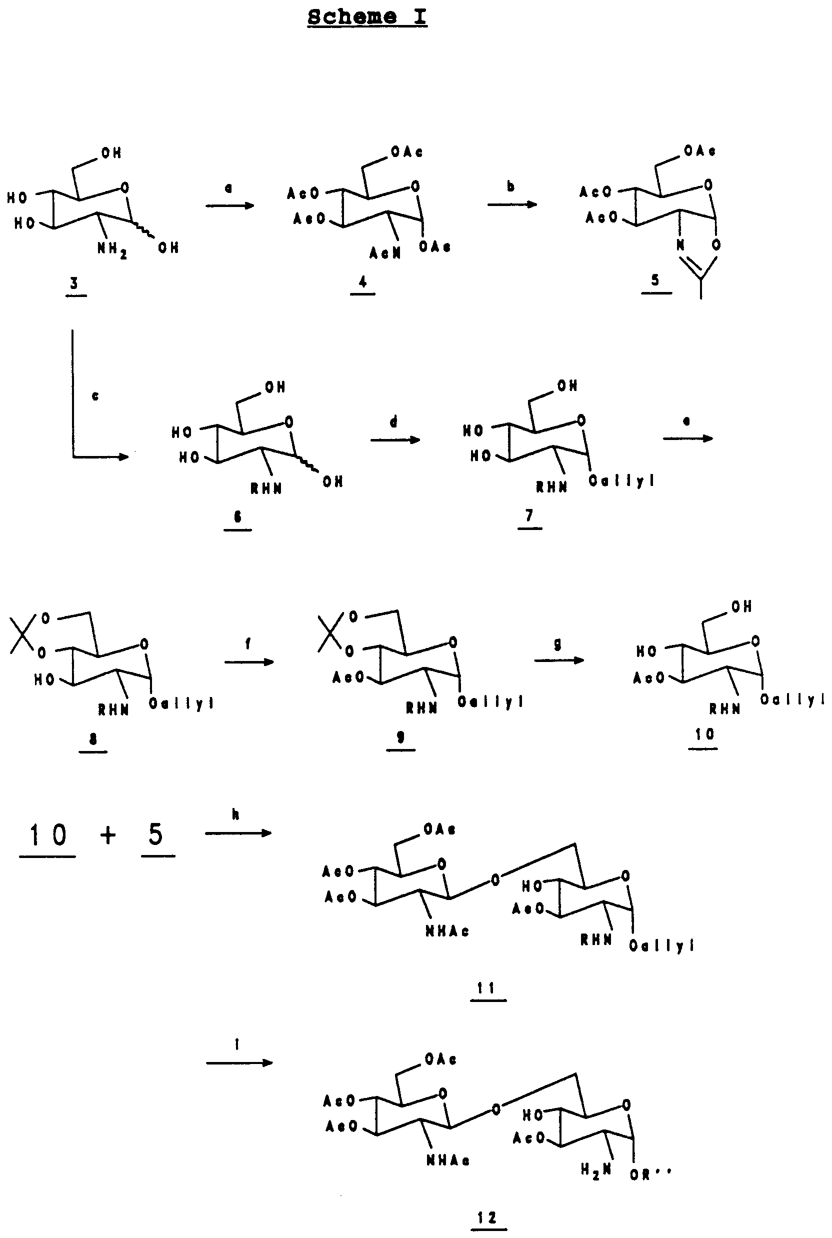

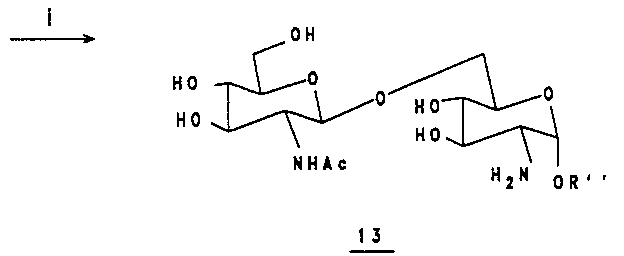

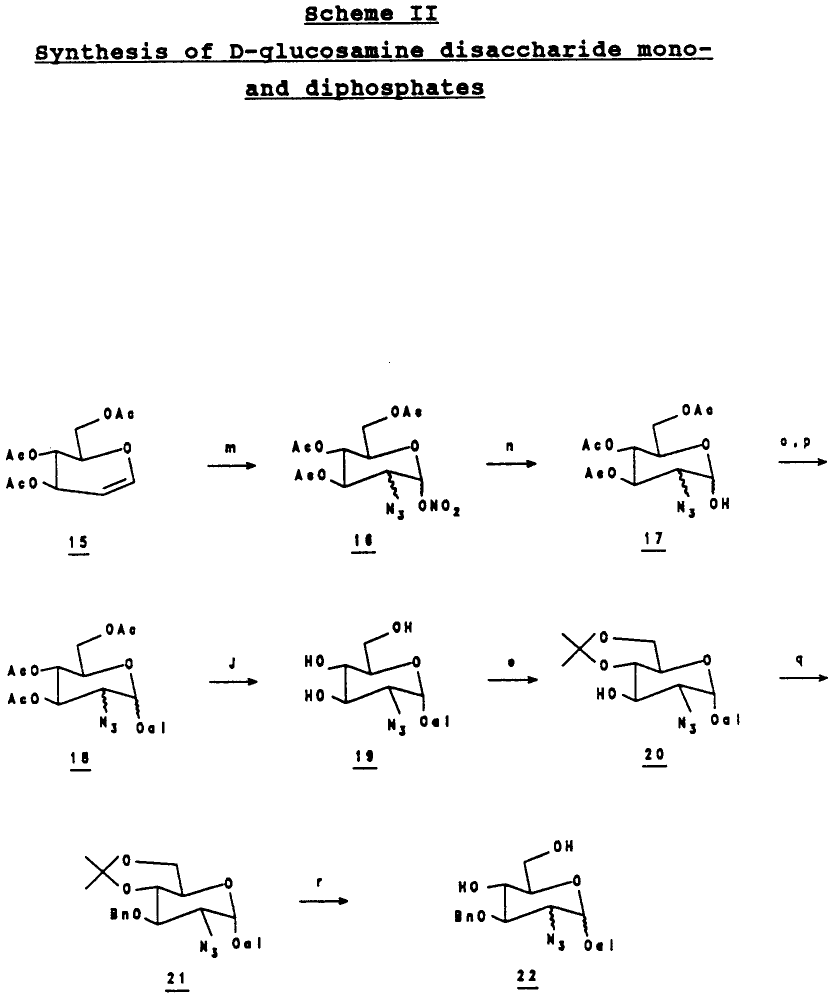

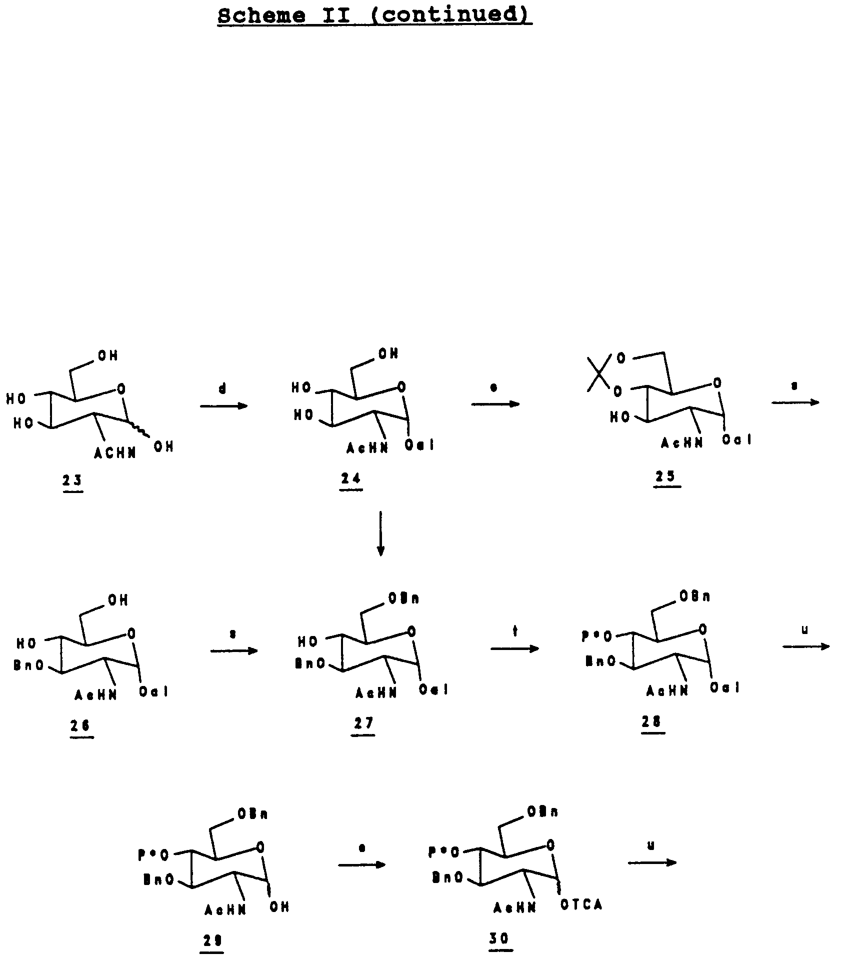

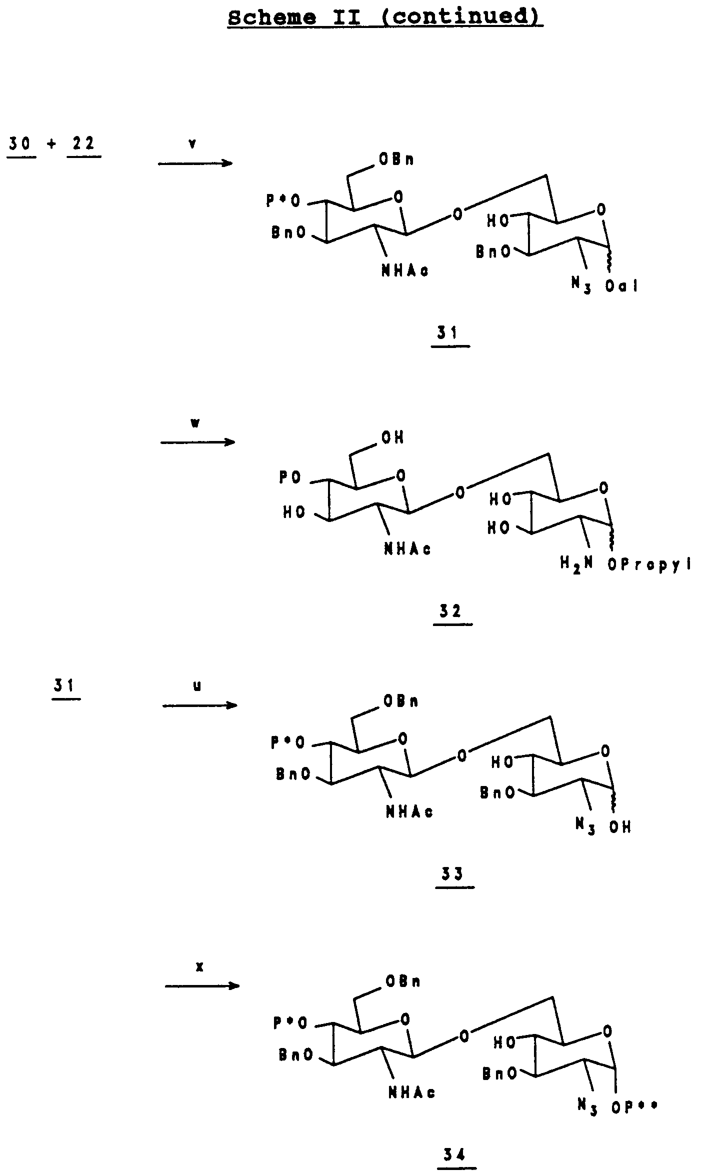

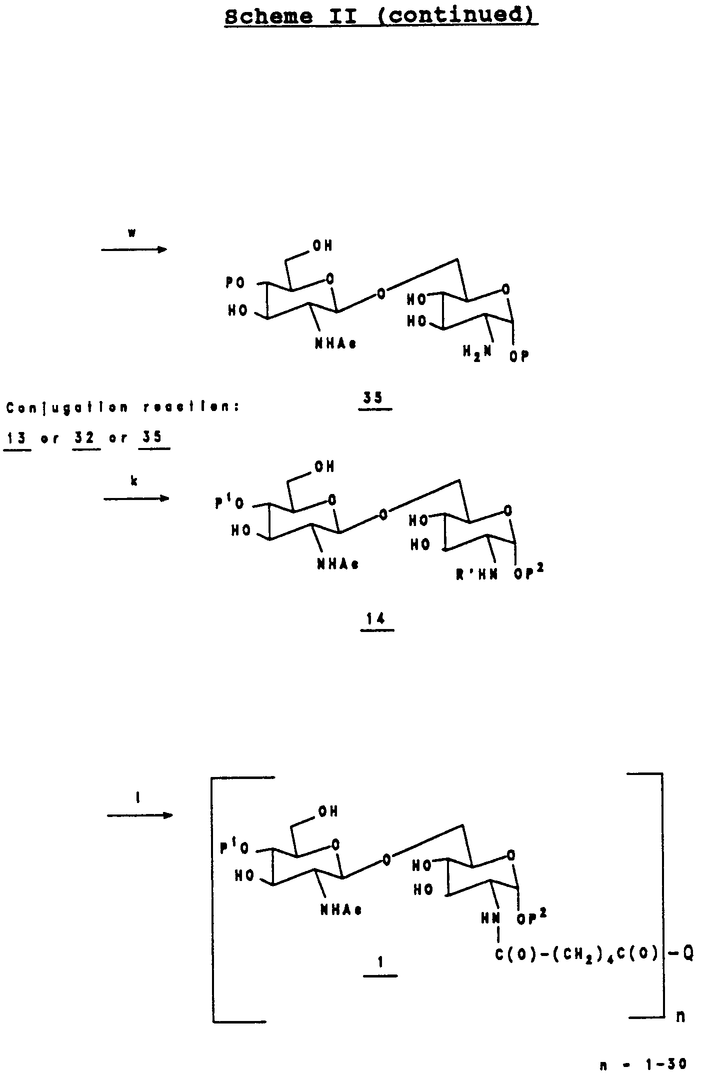

In the foregoing Scheme I and Scheme II,
R is benzyloxy carbonyl (CBZ) or 2,2,2 trichloroethyloxycarbonyl, R' is C(O)(CH₂)₄ C(O)O-X wherein
X is 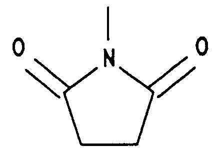
R'' is propyl;
al is allyl group;
Bn is benzyl;
P* is diphenyl phosphate;
TCA is a trichloroacetimidate group;
P** is dibenzyl phosphate;
P is phosphate;
P¹ is hydrogen or phosphate;
P² is propyl, allyl, phosphate or hydrogen;
The reagents used in the foregoing reactions may be as follows:
a. ACETICANHYDRIDE/PYRIDINE
b. TIN TETRACHLORIDE
c. BENZYLOXYCARBONYLCHLORIDE
d. ALLYLALCOHOL/HCL
e. DIMETHOXYPROPANE/p-TSA
f. ACETICANHYDRIDE/PYRIDINE
g. 90% ACETIC ACID, 100°C
h. P-TSA, CHLOROFORM
i. Pd/C, ETHANOL, CYCLOHEXENE
j. AMBERLITE IRA 401
m. cericammonium nitrate/sodium azide
n. sodium nitrite
o. potassium carbonate/trichloroacetonitrile
p. allylalcohol/trimethylsilyltriflate
q. benzylbromide/sodiumhydride
r. 7N Hcl, rt 1h
s. benzylbromide/barium hydroxide/barium oxide
t. diphenylphosphorochloridate/dimethylaminopyridine
u. 1,5-cyclooctadiene bis(methyldiphenylphosphine)-iridium hexafluorophosphate/iodine
v. molecular sieves/trimethylsilyltriflate
w. Pd/C, Pt₂O, H₂ 8 bar
x. butyllithium/dibenzylphosphorochloridate
k. and l. See the text.
However, other reagents may be suitable as known by those skilled in the art.

In accordance with Scheme I, the synthesis of propyl 6-O-[2-acetamido-2-deoxy-β-D-glucopyranosyl]-2-amino-2-deoxy-α-D-glucopyranoside(13) is achieved by the glycosidation of the donor 2-methyl-(3,4,6-tri-O-acetyl-1,2-dideoxy-α-D-glucopyrano)-[2,1-d]-oxazoline(5) and the novel acceptor allyl 3-O-acetyl-2-(benzyloxycarbonyl)amino-α-D-glucopyranoside(10).

The donor 2-methyl-(3,4,6-tri-O-acetyl-1,2-dideoxy-α-D-glucopyrano)-[2,1-d]-oxazoline(5) is prepared in two steps as described by Srivatsava V.K. (1982). In the first step, D-glucosamine hydrochloride is treated with acetic anhydride and pyridine to yield pentaacetate 4. The pentacetate is converted to oxazoline 5 by treating it with tin tetrachloride in a suitable solvent such as methylenechloride.

The novel acceptor allyl 3-O-acetyl-2-(benzyloxycarbonyl)amino-α-D-glucopyranoside(10) may be prepared in five steps starting from commercially available D-glucosamine hydrochloride. D-Glucosamine hydrochloride is treated with benzyloxycarbonylchloride or 2,2,2 trichloroethylchloroformate (Bergamann and Zervas, 1932) to give derivative(6). This reaction may be carried out in a suitable solvent such as water containing sodium bicarbonate. The amino-D-glucopyranose derivative(6) is then converted to the allyl 2-substituted-amino-α-D-glucopyranoside(7) by the reaction with allylalcohol-HCl. This intermediate has been reported in the literature (Sugawara, Tamio et al., 1989). When compound 7 is reacted with 2,2'-dimethoxypropane in presence of P-TSA (Evans et al., 1967), allyl-2-substituted-amino-4,6-O-isopropylidene-α-D-glucopyranoside(8) is obtained (two steps). The hydroxy group in position 3 of 8 is esterified with acetic anhydride and pyridine (Weber and Khorana, 1972) to yield allyl 2-substituted-amino-3-O-acetyl-4,6-O-isopropylidene-α-D-glucopyranoside(9). The 3-O-acetyl group may be substituted with any C₁-C₄ alkyl ester by substituting the appropriate reagent for acid anhydride. This compound is converted to acceptor 10 by acidic hydrolysis (Lewbart and Schneider, 1969) of the isopropylidene group.

The glycosidation reaction of 5 and 10 is carried out in a suitable solvent such as refluxing chloroform in presence of p-toluene sulfonic acid. This reaction gives the desired 1-->6 β- configurated disaccharide 11 (Allyl-2-deoxy-6-O-[2-deoxy-2-acetamido-3,4,6-tri-O-acetyl-β-D-glucopyranosyl]-2-(benzyloxycarbonyl)amino-3-O-acetyl-α-D-glucopyranoside).

The structure of this novel disaccharide was confirmed in experimental studies by ¹H-NMR and ¹³C-NMR. In ¹H-NMR, the coupling constant between H-1 and H-2 of the nonreducing moiety is 8.9 which is characteristic of a β-linkage. When 11 is hydrogenated on palladium/carbon (Bergamann and Zervas, 1932), the nitrogen protecting group is removed, and the allyl group reduced to propyl, the disaccharide 12 is produced. 12 is deacetylated to obtain the novel synthetic disaccharide propyl 6-O-[2-acetamido-2-deoxy-β-D-glucopyranosyl]-2-amino-2-deoxy-α-D-glucopyranoside(13).

While the foregoing description describes the preparation of the novel disaccharides of the present invention in accordance with Scheme I utilizing specific reagents, solvents and reaction condition, other variations in terms of solvents, reagents and reaction conditions are contemplated to be within the scope of the invention. For example, suitable solvents for the glycosidation reaction of 5 and 10 include carbon tetrachloride (CCl₄), dichloromethane (CH₂Cl₂), benzene and the like. Other variations will become apparent to those skilled in the art from the foregoing description.

### SCHEME II

### Synthesis Of D-Glucosamne Disaccharide Mono-And Diphosphates (32 And 35)

In accordance with the Scheme II, the synthesis of propyl 2-deoxy-6-[2-deoxy-2-acetamido-β-D-glucopyranosyl]-2-amino-D-glucopyranoside 4'-(phosphate) (32) and 2-deoxy-6-[2-deoxy-2-acetamido-β-D-glucopyranosyl]-2-amino-α-D-glucopyranose 1,4'-bis (phosphate) (35) is achieved by the glycosidation of the novel donor 2-Deoxy-2-acetamido-3,6-di-O-benzylglucopyranosyl trichloroacetimidate-4-O-diphenyl phosphate (30) and the novel acceptor allyl 2-azido-3-O-benzyl-D-glucopyranoside (22). The novel acceptor allyl 2-azido-3-O-benzyl-D-glucopyranoside (22) is synthesised in 8 steps starting from commercially available triacetyl glycal (15). Triacetyl glycal is subjected to azido-nitration as described in the literature (Grundler and Schmidt, 1984). This azido- nitration results in the mixture of compounds namely, α-D-gluco-azide (19%), β-D-glucoazide (30%) and α-D-manno-azide (30%). The ratios were determined from the integrals of the H-C(1) signals in the ¹H-NMR spectrum of the mixture. No attempt was made to separate these mixtures at this stage. The azido-nitrate 16 is treated with sodium nitrate in dioxane (Grundler and Schmidt, 1984) to give 17, which is converted to allyl glycoside 18 in two steps. Deacetylation of 18 to furnish 19 is achieved by treating 18 with Amberlite IRA 401 Resin. The compound 19 is treated with dimethoxypropane and p-toluene sulfonic acid (Evans et al., 1967). From this reaction product, 20 is isolated by flash chromatography. 20 is a mixture of α- and β- anomers. The structure of 20 was confirmed by ¹H-NMR and ¹³C-NMR spectra. 20 is converted to 22 in two steps, first benzylation (benzylbromide/sodium hydride, Czernecki et al., 1976) of hydroxy group at position 3 and then hydrolysis (Lewbart and Schneider 1969) of the 4,6-O-isopropyledene group. Other equivalents of benzyl may be used for the protection of the hydroxy group at position 3. Contemplated equivalents include substituted benzyl moieties where the substituents do not interfere with the function of the protecting group.

The novel donor 2-Deoxy-2-acetamido-3,6-di-O-benzyl-glucopyranosyl trichloroacetimidate-4-O-diphenyl phosphate (30) is prepared from commercially available N-acetyl glucosamine (23) in seven steps. First 23 is allylated and then subjected to isopropylidation (Evans et al., 1967) to yield 25. The structure of 25 was confirmed by ¹H-NMR and ¹³C-NMR spectra. 2-Deoxy-2-acetamido-4,6-O-isopropylidene-α-D-glucopyranoside (25) is subjected to benzylation in presence of barium oxide and barium hydroxide to yield 2-deoxy-2-acetamido-4,6-O-isopropylidene-3-O-benzyl-α-D-glucopyranoside, which is treated with 7N HCl to give 2-deoxy-2-acetamido-3-benzyl-α-D-glucopyranoside (26). 26 is subjected to the same benzylation conditions as described for 25 to furnish 2-deoxy-2-acetamido-3,6-O-dibenzyl-α-D-glucopyranoside (27). The compound 27 may also be prepared from 24 using two equivalents of benzylation reagents. As stated above for the protection of the hydroxy group of compound 20, other equivalents of benzyl may be used for the protection of the hydroxy group of compound 27. Phosphorylation of 27 using diphenyl phosphorochloridate in presence of dimethylamino pyridine in dry chloroform (Imoto M. et al., 1987) gives allyl 2-deoxy-2-acetamido-3,6-di-O-benzyl-α-glucopyranoside-4-O-diphenyl phosphate (28). In this reaction and the following glycosidation reaction of compound 30, it is important to use chloroform free of ethanol (HPLC grade chloroform containing 2-methyl 2-butene was used in these cases). 28 is converted to 29 by deprotection of the allyl protecting group using 1,5-cyclooctadiene bis(methyldiphenylphosphine)-iridium hexafluorophosphate and iodine (Imoto M. et al., 1987).

The donor 30 is obtained by treating 29 with potassium carbonate and trichloroacetonitrile (Schmidt R.R. and Grundler G., 1982). The disaccharide 31 is obtained by the glycosidation (Schmidt R.R. Angew. Chem. Int. Ed., 1986) of the acceptor 30 and the donor 22 in dry chloroform using trimethylsilyltriflate as a catalyst. The disaccharide 31 is subjected to hydrogenation using Pd/C and Pt₂O catalysts (Imoto M. et al., 1987) at 8 bar pressure to furnish 32.

The novel disaccharide 35 may be prepared from the novel disaccharide 31 in 3 steps, in the similar lines as reported by Imoto M. et al., (1987) for the synthesis of natural lipid A.

### 3. Conjugation of Disaccharide to Protein

Proteins which may be utilized according to the invention include any protein which is safe for administration to young mammals and which may serve as an immunologically effective carrier protein. In particular embodiments, cell surface proteins, membrane proteins, toxins and toxoids may be used. Criteria for safety would include the absence of primary toxicity and minimal risk of allergic reaction. Diphtheria and tetanus toxoids fulfill these criteria; that is, suitably prepared, they are non-toxic and the indicence of allergic reactions is acceptably low. Although the risk of allergic reaction may be significant for adults, it is minimal for infants. According to additional particular embodiments of the invention, appropriate carrier proteins include, but are not limited to, Salmonella flagellin, Hemophilus pilin, Hemophilus 15 kDa, 28-30 kDa, and 40 kDa membrane proteins, Escherichia coli heat labile enterotoxin LTB, cholera toxin, and viral proteins including rotavirus VO7 and respiratory syncytial virus F and G proteins.

In the "carrier effect" a weak antigen, by being attached to a stronger antigen as carrier (i.e. a heterologous protein), becomes more immunogenic than if it were presented alone. If an animal has been previously immunized with the carrier alone, the animal may be "primed" and produce an enhanced immune response not only to carrier antigen but also to attached hapten groups. Infants are routinely immunized with tetanus and diphtheria toxoids. Thus, they would be primed for subsequent presentation of a saccharide antigen conjugated to either of these toxoids as well as to the diphtheria cross-reactive protein CRM₁₉₇.

The carrier proteins to which the capsular polymer is conjugated nay be native toxin or detoxified toxin (toxoid). Also, by relatively recent mutational techniques, one may produce genetically altered proteins which are antigenically similar to the toxin yet non-toxic. These are called "cross reacting materials", or CRMs. CRM₁₉₇ is noteworthy since it has a single amino acid change from the native diphtheria toxin and is immunologically indistinguishable from it.

Conjugation of capsular polymer to native toxin may reduce toxicity, but significant toxicity may remain. Thus, further detoxification of protein toxins employs formalin, which reacts with free amino groups of the protein. Residual toxicity may still be a concern. Furthermore, spontaneous detoxification is possible with any particular lot of vaccine and remains an issue of concern with this approach.

Alternatively, native toxin may be detoxified with formalin to produce conventional toxoid before conjugation to capsular polymer. However, the prior formalin treatment reduces the number of free amino groups available for reaction with the reducing groups of the capsular polymer fragment. CRMs, thus, have significant advantages in that they have no inherent toxicity yet none of their amino groups are occupied by the formalin. A further advantage is that no biohazards exist in working with CRMs.

In the case of CRM₁₉₇, which is immunologically identical to native toxin, treatment with formalin (though there is no need to detoxify) greatly enhances the immunological response. It is thought that this is due to stabilization of the molecule against degradation by mechanisms of the body and/or aggregation by cross-linking (immunogenicity of particles increases with size).

For all of the above reasons, tetanus and diphtheria toxoids are prime candidates for carrier proteins, yet there are others which may also be suitable. Although these others may not have the history of safety found with diphtheria and tetanus, there may be other overwhelming reasons to use them. For instance, they may be even more effective as carriers, or production economics may be significant. Other candidates for carriers include toxins of pseudomonas, staphylococcus, streptococcus, pertussis and Escherichia coli.

In a specific embodiment of the invention, the Lipid A analog disaccharides may be linked to CRM₁₉₇ protein which has been purified as follows:
CRM₁₉₇, produced by the strain Corynebacterium diphtheriae, may be separated from culture medium by passing the bacterial culture through a Millipore membrane, precipitating protein from the filtrate, and purifying CRM₁₉₇ by ion exchange chromatography. Alternatively, substantially pure CRM₁₉₇ may be obtained by any method known in the art.

The disaccharide may be covalently linked to carrier protein in the presence of an organic solvent and, optionally, any other agent (such as a condensing agent) in order to promote the linkage of the free primary amino group of the disaccharide to the protein. The disaccharide may be covalently linked to the carrier protein using a variety of methodologies known in the art, in order to obtain the immunogenic-protein conjugates of the present invention. Coupling methods that may be used include the following:
1) - Reacting the amino-activated disaccharide with the carboxyl groups of the carrier protein, via carbodiimide activation at acid pH;
2) - Reacting the amino-activated disaccharide with the bromide group of a bifunctional spacer containing an aldehyde group which is further reacted with the amino groups of the carrier protein, via reductive amination utilizing a reducing agent like sodium cyanoborohydride;
3) - Reacting the amino-activated disaccharide with the amino groups of the carrier protein, via a bifunctional spacer containing two bromide groups;
4) - Reacting the amino-activated disaccharide with the N-hydroxysuccinimidyl ester of a bifunctional spacer containing a sulphydryl group which is further reacted with the sulphydryl groups of the carrier protein, via oxidation by air or by utilizing known oxidizing agents;
5) - Reacting the amino-activated disaccharide with the N-hydroxysuccinimidyl ester of a bifunctional spacer containing an N-maleimido group which is further reacted with the sulphydryl groups of the carrier protein.

In a specific, preferred embodiment of the invention, the disaccharide bearing the primary amino group may be covalently linked to free amino groups present on the carrier protein through the use of a bifunctional molecule, each functional group being capable of reaction with either the terminal amino group of the disaccharide and amino groups present in the structure of the carrier protein, such that the bifunctional molecule may serve to link together the oligosaccharide and the carrier protein. In a preferred embodiment of the invention, the bifunctional group is a diester, and is, more particularly, a diester of adipic acid, which has been shown to be associated with more efficient glycosylation of protein. In a preferred, specific embodiment of the invention, the disaccharide is reacted with a succinimidyl diester of succinic or, more preferably, adipic acid as follows:

As shown in Scheme II, the synthetic disaccharides 13, 32 or 35 are derivatized by transforming the primary amino group to monosuccinimidyl ester 14 by reaction with bis-succinimdyl ester of adipic acid. For this purpose, the disaccharide 13 is dissolved in dimethyl sulfoxide (DMSO) and reacted with bis-succinimidyl ester of adipic acid(1:1 mol/mol) at 37°C for about 2h. More than 90% of amino groups were derivatized to monosuccinimidyl esters in our experiments. The resulting compound 14, in DMSO is finally reacted with the carrier protein such as CRM₁₉₇ in 0.1M NaHCO3 solution, at a molar ratio of about 25:1 (disaccharide:protein) at a pH of about 8.0 and a temperature of 0-50°C, preferably about 37°C. The conjugate is then dialyzed for about 24h, against phosphate buffer solution (PBS, pH=7.2) containing 0.01% thimerosal as preservative.

The present inventor prepared conjugates in accordance with the procedure described above as described in the following examples. The characterization of the conjugate was performed by : a) analysis of the residual amino groups in the protein CRM₁₉₇ (25/40), b) identification of glucosamine residue after acidic hydrolysis of the conjugate, by high performance chromatography(HPLC) (amino acid analysis) and c) SDS-PAGE (sodium dodecylsulfate polyacrylamide gel electrophoresis) for molecular weight increase in respect to the native CRM₁₉₇.

### 4. Vaccine Formulation and Administration

Suitable carrier media for formulating a vaccine include sodium phosphate-buffered saline (pH 7.4) or 0.125M aluminum phosphate gel suspended in sodium phosphate-buffered saline at pH 6 and other conventional media.

Generally, vaccines containing from about 5 to about 100 µg, preferably about 10 to 50 µg of disaccharide, are suitable to elicit effective levels of antibody against the Lipid A analog in young warm-blooded mammals. Of course, the exact dosage would be determined by routine dose/response experimentation. The concentration of the glycoproteinic conjugates for the preparation of vaccines for children is comprised within the range of about 25 to 200 µg of disaccharide. Greater doses may be administered on the basis of body weight. Several small doses given sequentially would be expected to be superior to the same amount of conjugate given as a single injection.

The vaccines of the invention may be administered to warm-blooded mammals of any age and are especially adapted to induce active immunization against septic shock in young mammals caused by the release of Lipid A endotoxin released by the pathogens Escherichia coli, Neisseria meningitidis, Pseudomonas aeroginosa, Salmonella typhi, Neisseria gonorrheae, Bordetella pertussis, Klebsiella pneumoniae and the like.

According to the invention, vaccine may be delivered subcutaneously, intravenously, intramuscularly, intraperitoneally, orally, or intranasally. Vaccine may comprise glycoconjugate in soluble or microparticular form, or incorporated into microspheres or microvesicles, including liposomes.

### 5. Utility of Oligosaccharide Conjugate Vaccines

In preferred embodiments of the invention, glycoconjugate vaccines directed against LPS produced by pathogenic bacteria are used to protect individuals from developing septic shock caused by these agents. Particularly susceptible individuals who would benefit from such a vaccine include young children with immature immune systems, asplenic individuals, as well as any individual with a compromised immune system or chronic disease, particularly acquired immunodeficiency syndrome (A.I.D.S.), hematopoietic malignancy, diabetes, chronic heart disease, chronic pulmonary disease, and sickle cell anemia. The glycoconjugates of the invention, by virtue of their conjugation to a carrier protein, enhance the immunogenicity of the disaccharides they carry.

Thus, the glycoconjugates of the invention may be used in vaccinations to confer protection against septic shock caused by any bacteria which produce LPS including Escherichia coli, Neisseria meningitidis, Salmonella typhi, Klebsiella pneumoniae, and Pseudomonas aerugenosa, etc.

The lipid A glycoconjugate prepared as described above using CRM₁₉₇ as the carrier protein was tested in rabbits to determine the IgG immune response to CRM₁₉₇ and Lipid A/LPS. The results are set forth in Table I.

**Table I**

| Rabbit IgG Immune Response to CRM₁₉₇ and Lipid A/LPS Induced by the Glyco-Conjugate CRM₁₉₇-synthetic analog Lipid A (P0), as Analysed by Dot-Blot | | | | |
|---|---|---|---|---|
| | | CRM₁₉₇ carrier-protein | Lipid A | LPS |
| Rabbit #1 | Wk 0 | 0 | 0 | 0 |
| | Wk 3 | 1,600 | 100 | 50 |
| | Wk 6 | 6,400 | 200 | 200 |
| | Wk 8 | 12,800 | 200 | 200 |
| Rabbit #2 | Wk 0 | 0 | 0 | 0 |
| | Wk 3 | 800 | 0 | 0 |
| | Wk 6 | 6,400 | 50 | 50 |
| | Wk 8 | 12,800 | 100 | 100 |
| Rabbit #3 | Wk 0 | 0 | 50 | 100 |
| | Wk 3 | 400 | 200 | 200 |
| | Wk 6 | 3,200 | 200 | 200 |
| | Wk 8 | 6,400 | 400 | 400 |
| Rabbit #4 | Wk 0 | 0 | 50 | 50 |
| | Wk 3 | 800 | 100 | 100 |
| | Wk 6 | 6,400 | 200 | 200 |
| | Wk 8 | 12,800 | 200 | 200 |
| Antigen: CRM₁₉₇-synthetic analog Lipid A (P0). Ratio carbohydrate/protein: 0.1(w/w) or 15 (mol/mol) DOSE: CRM₁₉₇ 100 µg, Aluminum Hydroxide 1 mg/dose | | | | |

Upon further study of the specification and appended claims, further objects and advantages of this invention will become apparent to those skilled in the art.

This invention will be described in greater detail in conjunction with the following, non-limiting, specific examples.

### Example 1

### D-Glucosamine Pentaacetate (4)

To a solution of D-glucosamine hydrochloride (30g) in dry pyridine (180 ml), acetic anhydride was added at room temperature and the solution was stirred overnight. Methanol 20 ml was added and the solvents removed at 50°C. The residue was dissolved in EtOAc(400ml). This solution was washed with M HCl, water and brine. After drying over MgSO₄, solvent was removed to obtain crude 4 (44g) which was crystallized from EtOH. mp = 136-37°C. Rf 0.27 (EtOAc). ¹H-NMR: 6.08 (d, J = 3.3, H-1), 5.86 (d, J = 8.95, NH), 5.45 (m, 2H, H-3 and H-4), 4.38 (ddd, H-5), 4.17 ( dd, J - 12.5, 3.87, Hₐ-6, 3.95 (m, 2H, H_{b}-6, H-2) and 2.15-1.85 (4s, 5Ac).

### Example 2

### 2-Methyl-(3,4,6-tri-O-acetyl-1,2-dideoxy-α-D-glucopyrano) - [2,1-d]-2-oxazoline (DONOR,5):

To a solution of pentaacetate 4 ( 13g) in dry methylenechloride (250 ml), stannic tetrachloride ( 2.6g, 1.15ml) was added at room temperature. The resulting solution was stirred for 48h under nitrogen atmosphere. The reaction was neutralized by addition of 10% NaHCO₃, resulting precipitate was filtered through celite pad. The filtrate was washed with water, brine and dried (MgSO₄). After removing the solvent, the product was subjected to flash chromatography (FC) (2:1:1::Hexane:Ether:Acetone), to get 5 (3.2g) and unreacted 4(7 g). ¹H-NMR (CDCl₃, 300MHz): 5.98 ( d, J = 7.3 , H-1): 2.1-2.09 (3s, oAc). Rf.= 0.19 { Benzene :Ether:Methanol(7:7:0.5) }.

### Example 3

### 2-Deoxy-2-(benzyloxycarbonyl)amino-D-Glucose (6):

Benzyloxycarbonyl chloride (40 g, (39.3ml), 236 mmol) was added slowly to a cooled (0-4°C) solution of 2-amino-2-deoxy-D-glucose hydrochloride (34 g, 158 mmol) and sodium bicarbonate (34 g, 405 mmol) in water (500 ml). The mixture was stirred at this temperature for 2h and then at room temperature overnight. The colorless precipitate of 6 was collected by filtration, washed with water, ether and dried at high vacuo (25g, 50%). mp. 216-217°C(dec.). Rf = 0.31 (MeOH:EtOAc:: 1:9).

### Example 4

### Allyl 2-deoxy-2-(benzyloxycarbonyl) amino-α-D-Glucopyranoside (7):

Compound 6 (35 g) was heated with stirring at 100°C in allylalcohol containing 2 (W/V)% dry HCl for 0.5h. The mixture was cooled and the solvent evaporated in vacuo. The residue was dried by coevaporating with toluene (50 ml) three times. This compound was used in the next step without further purification. mp= 124-26°C. Rf = 0.47 7.05(d,J=7.8, NH), 6.0-5.8(m,CH=), 5.4-5.25(m,CH₂=), 5.05-5.15(bs, AB), 4.98 and 4.48(bs,2OH), 4.78(d,J=3.1, H(1)), 4.2-3.85(m, CH₂-allyl) 3.75-3.05(m,7H).

### Example 5

### Allyl 2-deoxy-4,6-O-isopropylidene-2(benzyloxycarbonyl) amino-α-D-Glucopyranoside (8):

The above dry product(5g) was dissolved in 2,2'dimethoxypropane ( 50 ml ). To this stirring solution, dry p-toluene sulfonic acid ( 100 mg) was added at room temperature. The reaction mixture was stirred overnight. Triethylamine (2ml) was added to the reaction mixture and the solvent was evaporated at reduced pressure. The residue was subject to FC (SiO₂ = 150 g, 5:1; CH₂Cl₂:Acetone containing 1% TEA) to yield 3.2 g (57%) of 8. mp = 107-109°C, Rf =0.54 (1:1::Hex:EtOAc).
¹H-NMR (CDCl₃, 300MHz): 1.42 and 1.50 (2s, 6H, CH₃); 2.75 (b, 1H, OH); 3.5-4.2 (m, 8H); 4.82 (d, J = 3.4, H-1); 5.1-5.3 (m, 5H, NH,CH₂-benzyl,CH₂=), 5.75-5.90( m, 1H, CH=) and 7.35(bs,5H,arom.).
¹³C-NMR: 157(s,O=C-O-benbyl); 136.75(s,arom.); 133.98(d,CH=); 129.0-128.86(d,arom.); 118.6(t,CH₂=); 100.5(s,isopr.); 97.94 (d,C(1)); 75.1 and 70.96(d); 69.1 (t, CH₂-O-benzyl); 67.83 (t, O-CH₂-allyl); 64.16(d); 62.84 (t); 59.32 (d,C(2)); 29.68 and 19.7 (q, CH₃).

### Example 6

### Allyl 2-deoxy-2-(benzyloxycarbonyl)amino-3-O-acetyl α-D-Glucopyranoside (10):

To a solution of 8 (9.00 g, 23 mmol) in dry pyridine(60ml), acetic anhydride(15ml) was added at room temperature. The reaction soluton was stirred overnight. Solvents were removed at 35°C under vacuo to yield allyl 2-deoxy-2-(benzyloxycarbonyl)amino-3-O-acetyl-4,6-O-isopropylidene α-D-Glucopyranoside (9). The acetylated product 9(5 g) was heated in 90% acetic acid at 95°C for 10 min. The solvent was evaporated in vacuo. The product (3.91 g, 85%) was a gummy material. Rf = 0.2 (Hex.:EtOAc;1:1).
¹H-NMR(CDCl₃):1.9 (s, CH₃); 2.56 (d,OH); 3.65-4.2 (m, 9H); 4.86 (d, J = 3.6Hz, H-1); 4.95-5.3 (m, 6H); 5.75-5.95 (m, 1H, CH=) and 7.35(bs,arom.).
¹³C-NMR: 172.67 (s,O=C-O-acetyl);
156.83(s,O=C-O-benzyl); 136.86(s,arom.); 133.93(d,CH=); 129.0-128.64(d,arom.); 118.69(t,CH₂=); 97.25 (d,C(1)); 74.6 and 72.39(d); 69.04(t, CH₂-O-benzyl; 68.83(d); 67.83 (t, O-CH₂-allyl); 61.89 (t); 54.33 (d,C(2)) and 21.40 (q, CH₃).

### Example 7

### Allyl 6-O-[2-deoxy-2-acetamido-3,4,6-tri-O-acetyl-β-D-glucopyranosyl]-2-deoxy-2-(benzyloxycarbonyl)-amino-3-O-acetyl-α-D-glucopyranoside(11):

The solution of donor 5 ( 0.32g, 0.10 mmol), acceptor 10 (0.8g, 0.20 mmol) and p-TsOH (40 mg) in dry chloroform (20 ml) was refluxed under N₂ atmosphere for 24h. After cooling, the solution was washed with 10% aq. NaHCO₃, water and brine. Solvent was removed and the residue subjected to FC (2:1:: EtOAc:Hex.) to obtain 11 (0.5g). Rf. = 0.35 (EtoAc).
¹H-NMR (CDCl₃, 300MHz): 1.90, 2.0, 2.05(s, CH₃); 3.49(d,OH); 3.55-4.29(m, 9H); 4.8(d, J=3.54, H-1); 4.89(d,J=8.42,H-1'); 4.95-5.35(m,5H); 5.74-5.89(m,CH=); 6.1(d,NH) and 7.25-7.35(m, arom.).
¹³C-NMR: 172.35, 171.94, 171.46, 170.1 (s,O=C-acetyl); 156.58 (s,O=C-O-benzyl; 136.91(s,arom.); 133.83(d,CH=); 129.13, 128.78,128.57(d, arom.); 118.72(d, CH₂=); 103.02 (d,C(1')); 101.68 (d,C(1)); 74.42, 72.73, 72.51, 71.43, 69.28, 68.96(d); 68,79 (t, CH₂-O-benzyl and CH₂-O-allyl)); 68.65(d); 67.44, 62.65(t, CH₂); 55.38, 54.31(d,C(2) and C(2')); 23.77 and 21.3(q, CH₃).

### Example 8

### Propyl 6-O-[2-deoxy-2-acetamido-3,4,6-tri-O-acetyl-β-D-glucopyranosyl]-2-deoxy-2-amino-3-O-acetyl-α-D-glucopyranoside(12):

To a solution of 11 (0.3g) in ethanol (16ml) and cyclohexene (6ml), palladium hydroxide was added and the resulting reaction mixture was refluxed for 2h. After filtration of the catalyst, the solvent was evaporated and the residue subjected to the FC (EtoAc) to yield 12 (130 mg, 60%). Rf. 0.54(EtOH:MeCN:H₂O::8: 8:4). mp. amorphous powder. ¹H-NMR: 0.94(t,CH₃); 1.6(q,CH₂); 1.9,2.0 and 2.1(s,CH₃C(O)); 3.25-4.3(m); 4.72-4.85(m); 4.8(d,J=8.3, h-1'); 4.92(d,J=3.12,H-1); 5.08(t,J=(.6,1H); 5.24(t,J=9.85,1H) and 5.9(NH).

### Example 9

### Propyl 6-O-[2-acetamido-2-deoxy-β-D-glucopyranosyl]-2-amino-2-deoxy-α-D-glucopyranoside(13):

The disaccharide 12(50mg) was deacetylated in methanol (10ml) using Amberlite IR-400(OH⁻) (0.5g) resin by stirring at rt for 4h. The resin was filtered and the filtrate concentrated to give propyl 6-O-[2-acetamido-2-deoxy-β-D-glucopyranosyl]-2-amino-2-deoxy-α-D-glucopyranoside(13), 30mg, 85%). Rf. mp. amorphous powder.
¹H-NMR(DMSO-D₆): 0.85(t,CH₃); 1.55(q,CH₂CH₃); 1.80(s,CH₃C(O)-);

### Example 10

### O-Nitro-3,4,6-tri-O-acetyl-2-azido-pyranose(16):

Under nitrogen atmosphere, finely powdered and dried ceric ammonium nitrate (60g) and sodium azide (4g) were added to a solution of glucal triacetate (15, 10g) in dry acetonitrile (200ml) at -20°C. Resulting suspension was vigorously stirred at this temperature for 7 h. Then the mixture was diluted with ether (300ml), washed with water, Na₂CO₃ solution, water and brine. After drying over MgSO₄, the solvents were removed to give 16 (12g) as a gummy material. 16 was dried at high vacuum pump and used in the next reaction without further purification. Rf.= 0.7(hexane:ethylacetate::1:1). ¹H-NMR: 2.0-2.15(s,CH₃CO), 4.0-4.3(m), 4.90-5.55(m), 6.33(d, J=4.2), 6.21(d, J=1.9) and 5.63 (d, J=8.9).

### Example 11

### 3,4,6-tri-O-acetyl-2-azido-pyranose(17):

To a solution of O-Nitro-3,4,6-tri-O-acetyl-2-azidopyranosides (16, 12g, from above experiment) in dioxane (70ml), a solution of sodium nitrite(14g) in water (15ml) was added, and the resulting mixture was heated at 80°C for lOh. After cooling to room temperature, ice cold water was added. Aqueous phase was extracted with ether and the ether extract was processed in the usual way to give the crude product 17. Crude 17 was subjected to FC (flash chromatography) (hexane:ethylacetate::l:l) to yield 3,4,6-tri-O-acetyl- 2-azido-pyranose (17, 7.5g, 62% in two steps).

### Example 12

### Allyl 2-azido-3,4,6-tri-O-acetyl-pyranoside (18):

To a stirring solution of 17 (4.6g) in dry dichloromethane, potassium carbonate (3.8g, flame dried) and trichloro acetonitrile (5.6ml) were added under dry nitrogen atmosphere. Stirred for 5h at room temperature. Insoluble potassium carbonate was filtered off, filtrate concentrated and dried at high vacuum to give trichloroacetimidate(6.6g). This trichloroacetimidate was take-up in dry chlroform(50ml) containing allylalcohol(4ml). To the resulting solution, trimethylsilyltriflate(O.lml) was added and the stirring under dry N₂ atmosphere was continued for 2h. Triethylamine (3ml) was added, and the resulting solution was concentrated and subjected to FC(dichloromethane:acetone::9.5:0.5) to give allyl 2-azido-3,4,6-tri-O-acetyl-pyranoside (18, 4.6g, 90%).

### Example 13

### Allyl 2-azido-pyranoside (19):

To the solution of 18(4.6g) in methyl alcoho1(300ml), Amberlite IRA-401 (OH-, lOOml) was added and stirred overnight. After filtration of the resin, the filtrate was concentrated to yield allyl 2-azido-pyranoside(19. 2g, 65%).

### Example 14

### Allyl 2-azido-4,6-O-isopropyledene-D-glucopyranoside (20):

Allyl 2-azido-pyranoside 2g) was homogenised with dimethoxy propane(50ml) using sonification. To this solution a catalytic amount of p-TSA was added and stirred overnight. The reaction mixture was taken-up in ethylacetate, after usual workup and concentration, the residue was subjected to FC (hexane:ethylacetate::3:1, containing 0.5% TEA) to yield 0.9g of allyl 2-azido-4,6-0-isopropyledene-D-glucopyranoside(20). Rf.=0.31(hexane:ethylacetate::3:1, containing 0.5% TEA).
lH-NMR: 1.40 and 1.50(s,CH₃), 3.10-4.20(m), 4.4(d, J=8.03, H-l of β-anomer), 4.90(d, J=3.74, H-l of α-anomer), 5.205.36(m,CH2=) and 5.9(m,CH=).
13C-NMR: 19.2 and 29.07(q,CH3), 97.70(d, C(l) of 100.06(d, C(l) of B), .101.55(s, C-CH3), 118.02(t, CH2=) and 133.31(d, CH=).

### Example 15

### Allyl 2-azido-3-0-benzyl-D-glucopyranoside (22):

The solution of allyl 2-azido-4,6-O-isopropyledene-D-glucopyranoside (20,130mg, 0.5mmol) in DMF(5ml) was treated with NaH(24mg, lmmol) and benzylbromide(171mg, 0.12ml,lmmol). Stirring continued for 3h, at this stage, thin layer chromatography (tlc) (hexane:ethylacetate::1:1) indicated complete formation of 21. This reaction mixture containing 21 was acidified with 7N HCl and stirred for 1h. Extraction with ether, usual workup and FC(hexane:ethylacetate::2:1) gave allyl 2-azido-3-O-benzyl-D-glucopyranoside 60mg, 40%).Rf. 0.17 (hexane :ethylacetate::1:1). 1H-NMR: 2.22 and 2.80 (b, 2 OH), 3.20-4.40(m), 4.75 and 4.95(AB, J=11.2, benzyl), 4.92(d,J=3.75, H-1 of α-) 5.2-5.4(m, CH2=) and 5.92(m, CH=). 13C-NMR: 62.6 and 62.75(t, C(6)), 63.63, 66.51(d), 69.18, 71.24(t,CH₂ of allyl), 70.78, 71.41 71.96(d), 75.74(t, benzyl), 80.67, 83.15(d), 97.53(d, C(l) of α-), 101.81(d, C(l) of β-), 118.63, 118.8(t, CH2=), 128.74, 129.31(d, arom), 133.78(d, CH=) and 138.6(s, arom).

### Example 16

### Allyl 2-deoxy-2-acetamido-α-D-glucopyranoside (24):

The suspension of N-acetylglucosamine(23, lg) in allylalcohol(lOml) containing 2% HCl was heated at lOO°C for 10 min. Allyl alcohol was evaporated at water pump and residue subjected to FC (EtOAc:EtOH::4:1) to yield Allyl 2-deoxy-2-acetamido-α-D-glucopyranoside (24, 0.9g, 76%). Rf. 0.32(EtOAc:EtOH::4:1), mp = 180-82°C. ¹H-NMR(DMSO-D₆): 1.85(s, CH₃), 3.10-3.70(m, 6H), 3.9 and 4.1(2dd, CH₂ of allyl), 4.68(d, J=3.0, H-1), 5.15 and 5.30(2bd, CH₂=), 5.9(m, CH=) and 7.74(d, J=7.75, NH).

### Example 17

### Allyl 2-deoxy-2-acetamido-4,6-O-isopropyledene-α-D-glucopyranoside (25):

Allyl 2-deoxy-2-acetamido-α-D-glucopyranoside (24, lg) was homogenised with dimethoxy propane(lOml) using sonification. To this solution a catalytic amount of p-TSA was added and stirred overnight. TEA(3ml) was added and solvents evaporated, the residue was subjected to FC (ethylacetate) to yield allyl 2-deoxy-2-acetamido-4,6-O-isopropyledene-α-D-glucopyranoside (25, lg, 86.5%). Rf. =0.5(ethanol:ethylacetate::1:9). lH-NMR: 1.40 and 1.50(2s, CH₃), 1.98(s,CH₃CO), 3.44(bs, OH), 3.58-4.20(m), 4.82(d, J=3.8, H-l), 5.22(dd, CH₂=), 5.85(m, CH=) and 6.07(d, J=8.7, NH). ¹³C-NMR: 19.71 and 29.69(q, CH₃), 23.88(q, CH₃CO), 54.68(d, C(2)), 62.82(t, C(6)), 64.15, 71.14, 75.29(d), 69.02(t, CH₂ of allyl), 97.65(d, C(l)), 100.5(s, C-CH₃), 118.69(t, CH2=), 133.99(d, CH=) and 172.08(s, CH₃C0).

### Example 18

### Allyl-2-deoxy-2-acetamido-3-0-benzyl-α-D-gluco pyranoside (26):

To a suspension of barium oxide(613mg), barium hydroxide(158mg) and 2-deoxy-2-acetamido-4,6-O-isopropyledene-α-D-glucopyranoside(25, 470mg) in dry DMF (lOml), benzylbromide was added slowly. Stirring continued for 3 h, when tlc showed no starting material, the reaction mixture was acidified with 7N HCl and stirred over night. Usual workup and FC (9:1, EtOAc:EtOH) gave ally1-2-deoxy-2-acetamido-3-O-benzyl-α-D-glucopyranoside(14, 210mg). Rf. 0.48(9:1, EtOAc:EtOH).lH-NMR:1.9(s,CH₃CO), 2.35 and 2.9(2s,OH), 3.5-4.3(m), 4.7(AB of benzyl), 4.8(d, J=3.7, H-l), 5.23(m), 5.85(m) and 7.3(m). ¹³C-NMR: 24.06(q,CH₃C0),52.63(d,C(2)), 62.84(t,C(6)), 68.83(t,CH2= of allyl), 71.34 and 72.18(d), 74.61(t, benzyl), 80.73(d), 97.55(d, C(1)), 118.45(t,CH₂=), 128.66 and 129.25(d), 134.1(d,CH=), 138.95(s,arom.) and 170.6(s,CH₃C0).

### Example 19

### Allyl 2-deoxy-2-acetamido-3,6-di-O-benzyl-α-glucopyranoside (27):

27 was prepared from 26 in the similar lines as described for 14 above. After acidification with 7N HCl, the reaction mixture was extracted with ethylacetate and the usual workup of the ethylacetate extract gave crude product which is a mixture of di- and tri-benzyl derivatives. From this mixture 27 was isolated by FC(4:1, CH²Cl₂:acetone) in 60%.
¹H-NMR:1.9(s,CH₃CO), 2.75(s,OH), 3.5-4.3(m), 4.5-4.78(2AB of benzyl), 4.82(d, J=3.72, H-l), 5.2(m), 5.4(d,J=9.28 NH), 5.85(m) and 7.3(m, arom). ¹³C-NMR: 24.06(q,CH₃CO), 52.42(d,C(2)), 68.82(t,C(6)), 70.78(t,CH₂= of allyl), 72.81 and 80.5(d), 74.31 and 74.48(2t, benzyl), 97.51(d, C(l)), 118.34(t,CH2=), 128.28, 128.4, 128.66, 129.1, 129.18 and 130.59(d), 134.22(d,CH=), 138.39 and 138.48(s,aromatic) and 170.44(s,CH₃CO).

### Example 20

### Allyl 2-deoxy-2-acetamido-3,6-di-O-benzyl-α-gluco pyranoside-4-O-diphenyl phosphate (28):

To a solution of 27(2.75g), dimethylaminopyridine(3g) and pyridine in dry chloroform(50ml) was added diphenyl phosphorochloridate(5ml) slowly. Stirring continued for 2h at room temperature. Reaction solution was diluted with chloroform and the usual workup and FC(9:1, methylenechloride:acetone), furnished 28 in 95% yield. Rf. 0.37 (dichloromethane:acetone::9:1). ¹H-NMR: 1.8(s, CH₃),3.66(dd), 4.05-3.76(m), 4.05-4.34(2AB), 4.85-4.72(m), 4.88(d, J=3.9), 5.6-5.0(m), 6.85(m, CH=) and 7.60-7.0(m, aromatic). ¹³C-NMR: 23.26(q,CH₃CO), 51.78(d,C(2)), 68.51, 70.25, 73.37, 73.54, 76.93, 78.11, 96.54(d, C(1)), 117.83(t,CH₂=), 120.14-128.69(arom), 133.46(d,CH=), 138.05 and 138.15(s,arom.) and 169.66(s,CH₃CO).

### Example 21

### 2-Deoxy-2-acetamido-3,6-di-O-benzyl-α-glucopyranose-4-O-diphenyl phosphate (29):

The solution of the compound 28(1.6g) and 1,5-cyclooctadiene bis(methyldiphenylphosphine)-iridiumhexafluorophosphate (0.2g) in dry oxygen free tetrahydrofuran(50ml) was stirred under hydrogen atmosphere till the orange color of the solution turns light yellow. Then hydrogen was replaced by dry nitrogen gas and stirring continued for 3h at 50°C. After cooling the reaction solution, iodine (0.5g) and water(10ml) were added and stirred overnight at rt. The reaction solution was diluted with ethylacetate(150ml) and the ethylacetate solution was washed with aqueous sodiumsulfite solution(5%), water and brine, dried over MgSO₄ and concentrated. The residue was subjected to FC(dichloromethane:acetone::4:1) to furnish the compound 29(0.75g, 50%).Rf. 0.12(dichloromethane:acetone::9:1). ¹³C-NMR: 23.17(q), 52.67, 68.8, 69.51, 69.61, 73.38, 73.53, 76.8, 77.25, 77.38, 77.49, 91.0(d, C(l)), 130.11-120.1(d, aromatic), 137.87, 138.03, 150.47, 150.51 and 170.49(all s).

### Example 22

### 2-Deoxy-2-acetamido-3,6-di-O-benzyl-glucopyranosyl trichloroacetimidate-4-O-diphenyl phosphate (30):

The mixture of the compound 29(0.53g), potassium carbonate(3g, flame dried) and trichloroacetonitrile(3ml) in dry chloroform(alcohol free) was stirred at rt overnight. Potassium carbonate was filtered off. The filtrate was concentrated, the residue(Rf. 0.65; dichloromethane:acetone::4:1) was dried under high vacuum and used as such in the next reaction.

### Example 23

### Allyl 6-O-[2-deoxy-2-acetamido-3,6-di-O-benzyl-β-D-glucopyranosyl]-2-deoxy-2-azido-3-O-benzyl-D-gluco pyranoside-4'-O-diphenylphosphate(31):

To a solution of the above trichloroacetimidate 30 and allyl 2-azido-3-O-benzyl-D-glucopyranoside 0.53g) in dry chloroform, molecular sieves (1Og, powdered, flame dried) was added. The solution was stirred under N₂ atmosphere for 1h. To this dry reaction mixture, trimethylsilyltriflate(0.2ml) was added slowly and stirring continued at rt for 6h. The reaction mixture was diluted with ethylacetate(100ml) containing triethylamine(3ml). The molecular sieves were filtered off and the filtrate was concentrated. FC(dichloromethane:acetone::4:1) of the residue gave the product 31 (0.15g),Rf. 0.49(dichloromethane:acetone::4:1). IR.(nujol): 2110cm⁻¹ (N₃). ¹H-NMR: 1.75(S,CH₃), 4.95-3.20(m), 5.40-5.04(m), 5.85(m) and 7.50-7.00(m, aromatic). ¹³C-NMR: 23.35(q,CH₃), 82.32-50.71(d, ring carbons), 96.89(d, α-anomeric C(1)), 100.45, 100.58 and 100.97( d, β-anomeric C(1') and C(1'), 117.95(t, CH₂=), 129.77-120.24(d, aromatic), 133.26(d, CH=), 137.96, 138.29, 150.41 and 171.89(all s).

### Example 24

### Propyl 6-O-[2-deoxy-2-acetamido-β-D-glucopyranosyl]-2-deoxy-2-amino-D-glucopyranoside-4'-0-phosphate(32):

The solution of the compound 31 (65mg) in methanol(15m1) was hydrogenated over 10% Pd/C(40mg) at 8 bar pressure for 2h. After addition of platinum oxide(20mg) catalyst to the reaction mixture, hydrogenation was continued at 8 bar for a further 2h. The catalysts were filtered off and the filtrate was concentrated to furnish the compound 32 (30mg).

### Example 25

### 6-O-[2-Deoxy-2-acetamido-3,6-di-O-benzyl-β-D-glucopyranosyl]-2-deoxy-2-azido-3-O-benzyl-D-glucopyranose-4'-O-diphenylphosphate(33):

This compound 33 was prepared from allyl 6-O-[2-deoxy-2-acetamido-3,6-di-O-benzyl-β-D-glucopyranosyl]-2-deoxy-2-azido-3-O-benzyl-D-glucopyranoside-4'-O-diphenylphosphate(31) by deprotection of allyl protecting group, in the similar lines as already reported for the preparation of 2-deoxy-2-acetamido-3,6-di-O-benzyl-α-glucopyranose-4-O-diphenyl phosphate (29).

### Example 26

### 2-Deoxy-6-[2-deoxy-2-acetamido-β-D-glucopyranosyl-2-amino-α-D-glucopyranose 1,4'-bis (phosphate) (35):

Compound 33 (80mg) was dissolved in dry THF(20ml) and cooled to -70°C under nitrogen atmosphere. The solution was treated with 1.6M butyllithium in hexane (0.1ml) and then dibenzyl phosphorochloridate (50mg). The mixture was stirred at -70°C for 5 min and 10% palladium/carbon (100mg) catalyst was added to it. The mixture was hydrogenated under 6 kgcm⁻¹ for 17h. After 17h, platinumoxide (100mg) was added and the hydrogenation was continued at 6 kgcm⁻¹ for further 2h. The catalysts were filtered off and the filtrate was neutralized with aqueous ammonia and concentrated to yield 35 mg of solid.

### References:

Bergamann M. and Zervas L., 65, 1192 (1932).
Czernecki S. et al., Tetrahedron Lett., 3535 (1976).
Evans M.E., Parrish F.W. and Long L., Jr., Carbohydr. Res., 3, 453 (1967).
Grundler G. and Schmidt R.R., Liebigs Ann Chem., 1826 (1984).
Imoto M. et al., Bull. Chem. Soc. Jpn., 60, 2205 (1987).
Lewbart M.L. and Schneider J.J., J. Org. Chem., 34, 3505 (1969).
Schmidt R.R. and Grundler G., Angew. Chem., 94, 790 (1982).
Schmidt R.R., Angew. Chem. Int. Ed., 25, 212 (1986).
Srivatsava V.K., Jr., Carbohydr. Res., 103, 287-292 (1982).
Sugawarw, Tamio et al., Jr., Carbohydr. Res., 194, 125-138 (1989).
Weber H., Khorana H.G., J. Mol. Biol. 72, 219 (1972).

## Claims

1. An immunogenic conjugate for the treatment of septic shock of the formula: 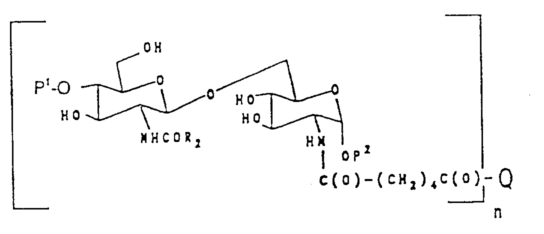 wherein
P¹ is hydrogen or phosphate;
R₂ is alkyl (C₁-C₄);
P² is hydrogen, propyl, allyl or phosphate;
n is an integer from 1-30; and
Q is a carrier protein or peptide.

2. An immunogenic conjugate according to claim 1 wherein the carrier protein or peptide is selected from CRM₁₉₇, a peptide derived from CRM₁₉₇, diptheria toxoid, tetanus toxoid and a peptide derived from diptheria or tetanus toxoid.

3. A compound of the formula: 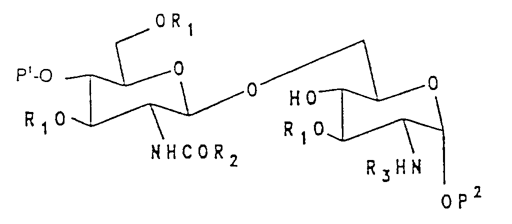 wherein
R₁ is hydrogen or acyl;
R₂ is (C₁-C₄) alkyl;
R₃ is hydrogen or CO(CH₂)₄-COOX wherein X is a moiety of the formula: 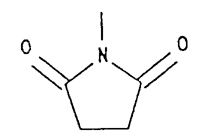
P¹ is hydrogen or phosphate and P² is hydrogen, propyl, allyl or phosphate.

4. A method of preparing an immunogenic conjugate which comprises (i) reacting a compound of the formula: 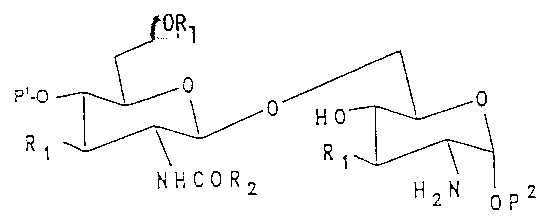 wherein R₁ is hydrogen or acyl, R₂ is alkyl(C₁-C₄), P¹ is hydrogen or phosphate and P² is hydrogen, propyl, allyl or phosphate with a molecule comprising two functional groups, one of which is capable of reacting with the primary amine of said compound and the other which is capable of reacting with a carrier protein; and (ii) reacting the product of (i) with said carrier protein such that conjugation occurs.

5. The method according to claim 4, in which the molecule comprising two functional groups of step (i) is a diester.

6. The method according to claim 4, in which the molecule comprising two functional groups of step (i) is a diester of adipic acid or a diester of succinic acid.

7. A covalent conjugate between a D-glucosamine disaccharide and a carrier protein produced by the method comprising the steps of:
(i) preparing an activated D-glucosamine disaccharide by reacting a compound of the formula: 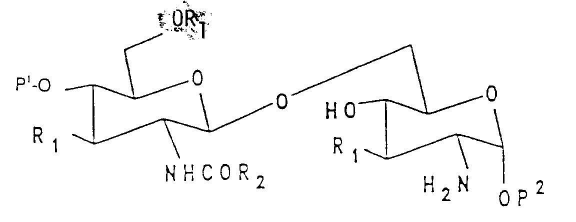 wherein R₁ is hydrogen or acyl, R₂ is alkyl (C₁-C₄), P¹ is hydrogen or phosphate and P² is hydrogen, propyl, allyl or phosphate, with a molecule comprising two functional groups, one of which is capable of reacting with the terminal group of the activated disaccharide and the other which is capable of reacting with said carrier protein; and (ii) reacting the activated D-glucosamine disaccharide product of (i) with said carrier protein such that conjugation occurs.

8. A vaccine for the prophylaxis of septic shock comprising a covalent conjugate of claim 1.

9. A vaccine for the prophylaxis of septic shock comprising a covalent conjugate prepared in accordance with claim 4.

10. A compound of the formula: 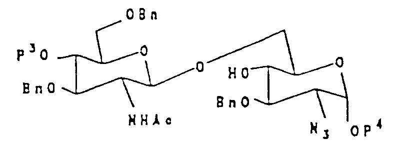 wherein Bn is benzyl; P³ is phosphate or diphenyl phosphate; P⁴ is allyl or propyl or hydrogen or dibenzyl phosphate; Ac is acyl.

## Patentansprüche

1. Immunogenes Konjugat für die Behandlung von septischem Schock der Formel: 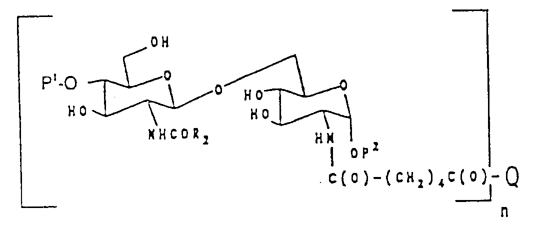 in der
P¹ Wasserstoff oder Phosphat ist;
R₂ (C₁-C₄)-Alkyl ist;
P² Wasserstoff, Propyl, Allyl oder Phosphat ist;
n eine ganze Zahl von 1 - 30 ist; und
Q ein Trägerprotein oder -peptid ist.

2. Immunogenes Konjugat nach Anspruch 1, in dem das Trägerprotein oder -peptid aus CRM₁₉₇, einem Peptid, das von CRM₁₉₇ abgeleitet ist, Diphteriatoxoid, Tetanustoxoid und einem Peptid, das von Diphteria- oder Tetanustoxoid abgeleitet ist, ausgewählt ist.

3. Verbindung der Formel: 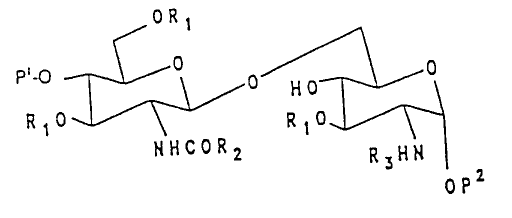 in der
R₁ Wasserstoff oder Acyl ist;
R₂ (C₁-C₄)-Alkyl ist;
R₃ Wasserstoff oder CO(CH₂)₄-COOX ist, worin X eine Einheit der Formel: 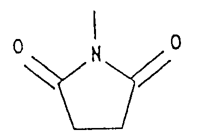 ist;
P¹ Wasserstoff oder Phosphat ist und P² Wasserstoff, Propyl, Allyl oder Phosphat ist.

4. Verfahren zur Herstellung eines immunogenen Konjugats, umfassend (i) das Umsetzen einer Verbindung der Formel: 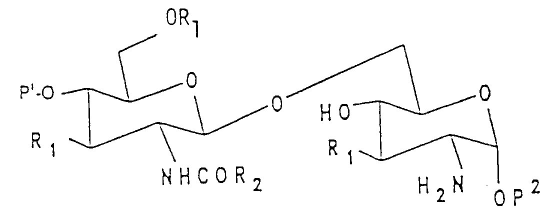 in der R₁ Wasserstoff oder Acyl ist, R₂ (C₁-C₄)-Alkyl ist, P¹ Wasserstoff oder Phosphat ist und P² Wasserstoff, Propyl, Allyl oder Phosphat ist, mit einem Molekül, das zwei funktionelle Gruppen umfaßt, von denen eine mit dem primären Amin der besagten Verbindung reagieren kann und die andere mit einem Trägerprotein reagieren kann; und (ii) das Umsetzen des Produkts von (i) mit besagtem Trägerprotein, so daß eine Konjugation stattfindet.

5. Verfahren nach Anspruch 4, in dem das Molekül von Schritt (i), das zwei funktionelle Gruppen umfaßt, ein Diester ist.

6. Verfahren nach Anspruch 4, in dem das Molekül von Schritt (i), das zwei funktionelle Gruppen umfaßt, ein Diester von Adipinsäure oder ein Diester von Bernsteinsäure ist.

7. Kovalentes Konjugat zwischen einem D-Glucosamindisaccharid und einem Trägerprotein, hergestellt durch das Verfahren, das die Schritte umfaßt:
(i) Herstellen eines aktivierten D-Glucosamindisaccharids durch Umsetzen einer Verbindung der Formel: 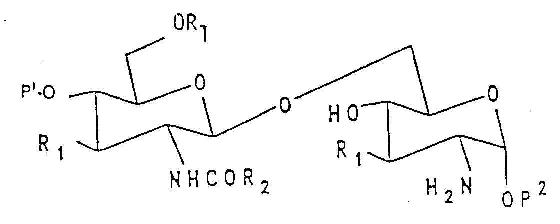 in der R₁ Wasserstoff oder Acyl ist, R₂ (C₁-C₄)-Alkyl ist, P¹ Wasserstoff oder Phosphat ist und P² Wasserstoff, Propyl, Allyl oder Phosphat ist, mit einem Molekül, das zwei funktionelle Gruppen umfaßt, von denen eine mit der endständigen Gruppe des aktivierten Disaccharids reagieren kann und die andere mit dem Trägerprotein reagieren kann; und
(ii) Umsetzen des aktivierten D-Glucosamindisaccharid-Produktes von (i) mit dem Trägerprotein, so daß eine Konjugation stattfindet.

8. Impfstoff für die Prophylaxe von septischem Schock, umfassend ein kovalentes Konjugat von Anspruch 1.

9. Impfstoff für die Prophylaxe von septischem Schock, umfassend ein kovalentes Konjugat, das gemäß Anspruch 4 hergestellt ist.

10. Verbindung der Formel: 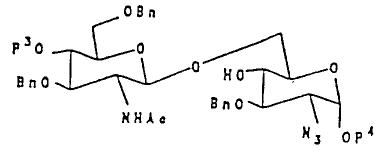 in der Bn Benzyl ist; P³ Phosphat oder Diphenylphosphat ist; P⁴ Allyl oder Propyl oder Wasserstoff oder Dibenzylphosphat ist; Ac Acyl ist.

## Revendications

1. Conjugué immunogène pour le traitement d'un choc septique, de formule : 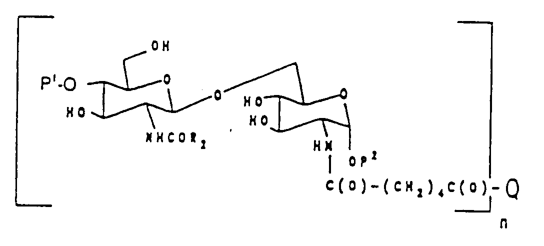 dans laquelle
P¹ représente un atome d'hydrogène ou un groupe phosphate ;
R₂ représente un groupe alkyle en (C₁-C₄) ;
P² représente un atome d'hydrogène, un groupe propyle, allyle ou phosphate ;
n représente un entier de 1 à 30 ; et
Q représente une protéine ou un peptide vecteur.

2. Conjugué immunogène selon la revendication 1, dans lequel la protéine ou le peptide vecteur, est choisi parmi la CRM₁₉₇, un peptide dérivé de la CRM₁₉₇, l'anatoxine diphtérique, l'anatoxine tétanique, et un peptide dérivé de l'anatoxine diphtérique ou tétanique.

3. Composé de formule : 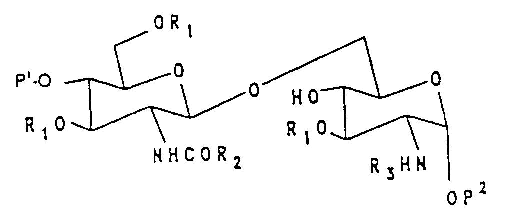 dans laquelle
R₁ représente un atome d'hydrogène ou un groupe acyle ;
R₂ représente un groupe alkyle en (C₁-C₄) ;
R₃ représente un atome d'hydrogène ou un groupe CO(CH₂)₄-COOX, dans lequel X représente un radical de formule : 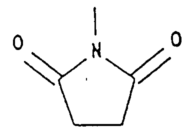
P¹ représente un atome d'hydrogène ou un groupe phosphate, et P² représente un atome d'hydrogène, un groupe propyle, allyle ou phosphate.

4. Procédé de préparation d'un conjugué immunogène, selon lequel (i) on fait réagir un composé de formule : 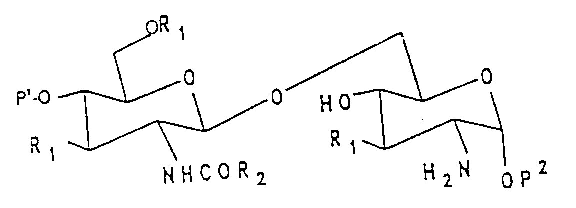 dans laquelle R₁ représente un atome d'hydrogène ou un groupe acyle, R₂ représente un groupe alkyle en (C₁-C₄), P¹ représente un atome d'hydrogène ou un groupe phosphate, et P² représente un atome d'hydrogène, un groupe propyle, allyle ou phosphate, avec une molécule comprenant deux groupes fonctionnels, l'un d'entre eux étant capable de réagir avec le groupe amine primaire dudit composé, et l'autre étant capable de réagir avec une protéine vecteur ; et (ii) on fait réagir le produit obtenu en (i) avec ladite protéine vecteur de façon à ce qu'une conjugaison ait lieu.

5. Procédé selon la revendication 4, dans lequel la molécule comprenant deux groupes fonctionnels de l'étape (i), est un diester.

6. Procédé selon la revendication 4, dans lequel la molécule comprenant deux groupes fonctionnels de l'étape (i), est un diester de l'acide adipique ou un diester de l'acide succinique.

7. Conjugué covalent entre un disaccharide de D-glucosamine et une protéine vecteur, préparé selon le procédé comprenant les étapes consistant :
(i) à préparer un disaccharide de D-glucosamine activé, en faisant réagir un composé de formule : 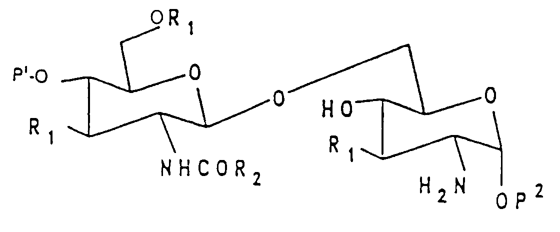 dans laquelle R₁ représente un atome d'hydrogène ou un groupe acyle, R₂ représente un groupe alkyle en (C₁-C₄), P¹ représente un atome d'hydrogène ou un groupe phosphate, et P² représente un atome d'hydrogène, un groupe propyle, allyle ou phosphate, avec une molécule comprenant deux groupes fonctionnels, l'un d'entre eux étant capable de réagir avec un groupe terminal du disaccharide activé, et l'autre étant capable de réagir avec ladite protéine vecteur; et
(ii) à faire réagir le dérivé activé du disaccharide de D-glucosamine obtenu en (i) avec ladite protéine vecteur de façon à ce qu'une conjugaison ait lieu.

8. Vaccin pour la prophylaxie d'un choc septique, comprenant un conjugué covalent de la revendication 1.

9. Vaccin pour la prophylaxie d'un choc septique, comprenant un conjugué covalent préparé selon la revendication 4.

10. Composé de formule : 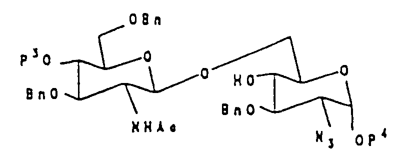 dans laquelle Bn représente un groupe benzyle ; P³ représente un groupe phosphate ou diphénylphosphate ; P⁴ représente un groupe allyle, propyle, un atome d'hydrogène ou un groupe dibenzylphosphate ; Ac représente un groupe acyle.
